# EUROPÄISCHE PATENTSCHRIFT

(11) **EP 2 278 929 B1**
(45) Veröffentlichungstag und Bekanntmachung des Hinweises auf die Patenterteilung: **04.01.2012**
(21) Anmeldenummer: 09749589.9
(22) Anmeldetag: 15.05.2009
(51) Int. Cl.: A61B 17/34

(54) **TORAX-TROKAR**
THORACIC TROCAR
TROCART THORACIQUE

(30) Priorität: 23.05.2008 DE 102008024900
(43) Veröffentlichungstag der Anmeldung: 02.02.2011
(73) Patentinhaber: Reuter, Alexander, 36211 Alheim (DE); Griesser-Aleksic, Aleksandar, 97616 Bad Neustadt a. d. Saale (DE)
(72) Erfinder: Reuter, Alexander, 36211 Alheim (DE); Griesser-Aleksic, Aleksandar, 97616 Bad Neustadt a. d. Saale (DE)
(74) Vertreter: Prüfer & Partner GbR European Patent Attorneys
(86) Internationale Anmeldenummer: PCT/EP2009/003478
(87) Internationale Veröffentlichungsnummer: WO 2009/141099

(56) Entgegenhaltungen:
- WO-A-94/04206
- US-A- 5 171 245
- US-A- 5 387 197
- US-A- 5 676 683
- US-A- 5 807 402
- US-A- 6 017 356

## Beschreibung

Die vorliegende Erfindung betrifft einen Torax-Trokar, umfassend einen in einer Gehäuseeinrichtung axial beweglich gelagerten Dom, wobei der Dorn eine abnehmbare Hülse aufweist.

Trokare sind aus dem Stand der Technik hinlänglich bekannt. So ist beispielsweise aus der US 5,674,237 ein sogenannter "Sicherheits-Trokar" bekannt, der an seinem vorderen Ende eine Schneide aufweist, wobei die Schneide durch einen sogenannten Sicherheitsschild umgeben ist. In dem Moment, wo der Trokar mit dem Sicherheitsschild auf die Bauchdecke gelangt, weicht der Schild entgegen der Kraft einer Feder zurück, und gibt insofern die Schneide frei, um dann mit Hilfe der Schneide die Bauchdecke durchstoßen bzw. durchschneiden zu können. Erfährt der Sicherheitsschild keinen Widerstand mehr, rückt der Sicherheitsschild wieder nach vorne über die Schneide und deckt insofern die Schneide ab.

In der DE 41 16 648 C2 ist ebenfalls ein Trokar beschrieben, wobei der Trokar einen hohlen Dorn mit einer am distalen Ende angeordneten Spitze aufweist, wobei in dem Dorn eine Schneideinrichtung längs verfahrbar ist. Die Schneideinrichtung besteht aus einem scheibenartigen rotierenden Messer, mit Hilfe dessen die Bauchdecke durchschnitten werden soll. Wie bereits ausgeführt, ist die Schneideinrichtung in dem hohlen Dorn des Trokars verschieblich angeordnet. Die Schneideinrichtung tritt aus dem vorderen Ende des Dornes nur dann heraus, wenn am hinteren Ende gegen die Kraft einer Feder auf die Schneideinrichtung Druck ausgeübt wird. In dem Moment, wo der Druck nachlässt, fährt die Schneideinrichtung aufgrund der auf sie einwirkenden Federkraft zurück in das Innere des Domes.

Aus der EP 0 265 193 B1 ist ein weiterer Trokar bekannt, bei dem eine axial bewegliche Schutzhülle aus Aluminium vorgesehen ist, die im Ausgangszustand über den Dorn des Torkars vorsteht. Sowohl der Dorn als auch die Schutzhülle sind in einem Gehäuse gelagert, wobei dieses Gehäuse ebenfalls die bereits zuvor erwähnte Feder aufweist, gegen die die Schutzhülle axial beweglich gelagert ist. Des Weiteren ist ein weiteres Gehäuse vorgesehen, durch das der Trokar-Dorn mit der Schutzhülle ragt. Dieses weitere Gehäuse besitzt eine Öffnung, die durch eine Rückschlagklappe verschließbar ist, wenn der Trokar-Dorn mit der Schutzhülle zurückgezogen worden ist. Dieses weitere Gehäuse weist darüber hinaus im Bereich eines Mundstückes eine Führungshülse auf, die die Schutzhülle für den Trokar-Dorn aufnimmt. Die Funktionsweise dieses Trokars lässt sich kurz wie folgt erklären:

Sobald die Schutzhülle auf die zu durchstoßende Bauchdecke trifft, weicht diese Schutzhülle gegen die Kraft der Feder zurück und gibt die Dornspitze des Trokars frei. In dem Moment, wo auf die Schutzhülle im distalen Bereich ein Druck nicht mehr ausgeübt wird, wird sich diese Schutzhülle aufgrund der Federkraft über den Dorn stülpen und die Dornspitze insofern abdecken. Wird der Trokar, also der Dom, mit der Schutzhülle aus der Öffnung herausgezogen, dann verbleibt zunächst das weitere Gehäuse am Körper des Patienten, wobei dann die Rückschlagklappe den Durchgang für den Dorn und die Schutzhülle verschließt.

Gegenstand der Erfindung ist ein sogenannter Torax-Trokar. Ein Torax-Trokar ist ein solcher, der bei einer Verletzung des Troraxbereichs durch den Brustkorb, d. h. durch die Rippen des Brustkorbes hindurch in den Brustraum hineingeschoben wird. Bei Unfällen kann es immer wieder vorkommen, dass es durch eine starke Prellung des Brustkorbes zu einem Zusammenfallen der Lunge kommt. Häufig auch sammelt sich im Brustraum Flüssigkeit und Luft an, die ebenfalls dafür sorgt, dass sich die Lunge nicht ausdehnen kann. In einem solchen Fall muss zügig gehandelt werden, und zwar mit dem Ziel, im Brustkorb einen Unterdruck aufzubauen, um dafür zu sorgen, dass sich die Lunge wieder ausdehnen kann.

Nun ist bereits ein Torax-Trokar bekannt, der einen Dorn umfasst, der von einer Hülse umgeben ist, wobei der Dorn am distalen Ende über die Hülse übersteht. Wie bereits erläutert, wird der Brustraum des Patienten durchstoßen, d. h. der Dorn wird beispielsweise zwischen zwei Rippen in den Brustkorb geschoben. Hierbei folgt die Hülse dem Dorn, wobei dann, wenn der Trokar die gewünschte Stelle im Brustraum mit dem vorderen Ende erreicht hat, der Dorn aus der Hülse herausgezogen wird. Alsdann kann ein Vakuum angelegt werden mit dem Ziel, die Ausdehnung der Lunge durch Ableitung von Luft und Flüssigkeit zu bewirken.

Es hat sich nun allerdings herausgestellt, dass die Verletzungsgefahr insbesondere für die Lunge, aber auch für andere im Brustraum befindliche Organe erheblich ist, wenn mit der Dornspitze voran der Trokar an die entsprechende Stelle im Brustraum geführt wird. Andererseits muss sichergestellt sein, dass die Dornspitze während des Durchstoßens durch den Brustkorb nicht zurückweichen kann. Ebenfalls muss sichergestellt sein, dass die Hülse mit dem Dorn zusammen durch den Brustkorb hindurch gelangt. Weiterhin soll sichergestellt sein, dass dann, wenn der Dorn den Brustkorb durchstoßen hat, die Dornspitze sich tatsächlich innerhalb der Hülse befindet, also nicht mehr über das distale Ende der Hülse vorsteht. Dies auch dann, wenn wiederum Druck auf das vordere, distale Ende der Hülse ausgeübt wird, wenn beispielsweise die Hülse auf innere Organe trifft.

Aus der WO 9404206 ist ein Trokar bekannt, worauf: die zweiteilige Form des Anspruchs 1 basiert.

Die der Erfindung zugrunde liegende Aufgabe besteht darin, einen Torax-Trokar bereitzustellen, bei dem sichergestellt ist, dass nach Durchdringen des Brustkorbes bei weiterer Führung des Trokars im Brustraum nicht die Gefahr der Verletzung innerer Organe besteht.

Insofern ist bei einem Torax-Trokar der eingangs genannten Art erfindungsgemäß vorgesehen, dass der Dorn eine aufnehmbare Hülse aufweist, wobei der Dorn relativ zu der Hülse in mindestens einer Stellung in der Gehäuseeinrichtung durch eine Arretiereinrichtung arretierbar ist, wobei in einer ersten arretierten Stellung der Dorn mit seiner Spitze über die Hülse übersteht, und wobei in einer zweiten Stellung der Dorn durch die Hülse überstehend überdeckt wird. Hieraus wird deutlich, dass der Dorn mit seiner Spitze während des Durchstoßens des Brustkorbes in jedem Fall über die Hülse vorsteht. Unmittelbar nach Durchstoßen des Brustkorbes wird durch Betätigen der Arretiereinrichtung dafür gesorgt, dass der Dorn aus dieser ersten Stellung in eine zweite Stellung übergeht, in der die Dornspitze durch die Hülse überstehend überdeckt ist. Das heißt, dass nach Durchstoßen des Brustkorbes und bei weiterer Führung des Trokars im Brustraum in keinem Fall mehr die Gefahr besteht, dass durch den Dorn innere Organe verletzt werden.

Des Weiteren ist der Dorn in der zweiten, zurückgezogenen Stellung in der Gehäuseeinrichtung arretiert, was bewirkt, dass der Dorn des Trokars auch in seiner zurückgezogenen Stellung fixiert ist, mithin nicht die Gefahr besteht, dass er- aus welchen Gründen auch immer -aus der Hülse herausgelangt und wiederum über die Hülse übersteht.

Vorteilhafte Merkmale und Ausführungsformen vergeben sich aus den Unteransprüchen.

So ist insbesondere vorgesehen, dass die Hülse aus elastisch nachgiebigem Material besteht, d. h. eine solche Hülse in gewissem Sinne durchaus steif, aber dennoch elastisch, so dass durch die Hülse selbst keine gefahr der Verletzung innerer Organe besteht.

Im Einzelnen kann die Arretiervorrichtung nach einem besonderen Merkmal derart ausgebildet, sein dass diese einen Arretierstift umfasst, der einen in Längsrichtung verlaufenden Schlitz aufweist, wobei der Schlitz zwei übereinander angeordnete unterschiedliche Durchmesser zeigt, wobei der Dorn zwei beabstandet zueinander angeordnete Nuten zur Erzeugung jeweils eines zum Ausgangsdurchmesser des Dorns unterschiedlichen reduzierten Durchmessers aufweist, wobei der im Schlitz angeordnete untere Durchmesser mit dem Durchmesser der Nuten korreliert und der Ausgangsdurchmesser des Dorns mit dem oberen Durchmesser im Schlitz. Der Arretierstift ist hierbei vorteilhaft in dem Gehäuse der Gehäuseeinrichtung unter der Last einer Feder angeordnet. Hieraus wird deutlich, dass dann, wenn der obere Durchmesser des Arretierstiftes in Eingriff mit dem Dorn gerät, der Dorn axial beweglich in dem Gehäuse der Gehäuseeinrichtung beweglich ist. Gelangt der Dorn mit seiner Nut in den Bereich des Arretierstiftes, wird der Dorn durch den Arretierstift gefangen, und zwar durch den unteren Durchmesser im Arretierstift, der dem Durchmesser der Nuten entspricht. Der Arretierstift steht - wie bereits ausgeführt - unter der Last einer Feder; diese Feder bewirkt, dass der Arretierstift in seiner Ausgangsstellung immer eine Stellung derart zum Dorn einnimmt, dass der Dorn im Bereich seiner Nut durch den Arretierstift gefangen gehalten wird.

Nach einem weiteren Merkmal kann vorgesehen sein, dass der Dorn in dem Gehäuse der Gehäuseeinrichtung entgegen der Kraft einer Dornfeder aus dem Gehäuse der Gehäuseeinrichtung herausziehbar ist. Hierzu ist im Einzelnen weiterhin vorgesehen, dass die Dornfeder sich axial einerseits an einem Bund in dem Gehäuse der Gehäuseeinrichtung und andererseits an einem endseitig am Dorn angeordneten Teller abstützt. Das Gehäuse der Gehäuseeinrichtung ist des Weiteren endseitig geschlossen, so dass der Teller des Dorns in der in der Hülse eingerückten Position des Dorns genau an dem Gehäuseverschluss anliegt. In dieser Position wird genau die zweite Nut im Dorn durch den Arretierstift gehalten. Das heißt, der Dorn ist hierbei durch die Nut in Verbindung mit dem Arretierstift in dieser Stellung, d. h. gegenüber dem stirnseitigen Ende der Hülse zurückgezogenen Ende, fixiert.

Die Funktionsweise des Torax-Trokars stellt sich nunmehr wie folgt dar:

Vor Ansetzen des Torax-Trokars an den Brustkorb wird unter Betätigung des Arretierstiftes, d. h. bei Herunterdrücken des Arretierstiftes, der Dorn per Hand axial entgegen der Kraft der Feder, die sich einerseits am Bund in dem Gehäuse und andererseits am Teller des Dorns abstützt, herausgezogen, und zwar so weit, bis die eine hintere Nut in den Bereich des Arretierstiftes gelangt. In dieser Stellung wird der Dom des Trokars durch den Arretierstift fixiert. Diese Stellung des Dorns ist üblicherweise bereits nach der Montage des Trokars vorgesehen. Alsdann wird der Trokar, d. h. der Dorn, mit der aufgesetzten Hülse durch den Brustkorb gestoßen, um dann unmittelbar nach dem Durchstoßen des Brustkorbes durch Betätigung des Arretierstiftes, d. h. durch Herunterdrücken des Arretierstiftes, den Dorn zurückschnellen zu lassen, d. h. in eine eingefahrene Position zu bringen, in der der Dorn mit seiner Spitze nicht mehr über die Hülse übersteht. In dieser Stellung ist der Dorn ebenfalls durch die zweite Nut durch den Arretierstift fixiert. Der Trokar kann dann ohne die Gefahr der Verletzung anderer innerer Organe weiter im Brustraum bewegt werden.

Nach einem weiteren Merkmal kann vorgesehen sein, dass die Hülse an dem der Gehäuseeinrichtung zugeordneten Ende einen Anschlusskörper mit mindestens einem Durchlass aufweist, wobei der Durchlass durch eine schwenkbare Klappe verschließbar ist. Wie bereits an anderer Stelle erläutert wird dann, wenn sich der Trokar an der gewünschten Stelle im Brustraum befindet, der Dorn zurückgezogen mit der Folge, dass die Hülse mit dem Anschlusskörper im Körper des Menschen verbleibt, wobei der Anschlusskörper aus dem Brustkorb herausragt. Wie bereits an anderer Stelle erläutert, befindet sich in dem Anschlusskörper eine schwenkbare Klappe, die federbelastet ist, so dass durch die Klappe der Durchlass des Anschlusskörpers automatisch verschließbar ist. Der Anschlusskörper besitzt im Bereich des Durchlasses nach einem weiteren vorteilhaften Merkmal einen Stutzen, der der Aufnahme einer Spritze dient. Hierbei weist die Spritze vorteilhaft ein Mundstück zur Aufnahme durch den Stutzen auf, wobei im Bereich des Mundstückes der Spritze ein hohler Anschlussdorn vorgesehen ist, der bis in den Anschlusskörper ragt und die Klappe aufstößt. Ist die Spritze in solcher Weise an dem Anschlusskörper befestigt, kann mit Hilfe der Spritze Flüssigkeit oder Luft aus dem Brustkorb abgesaugt werden. Es hat sich herausgestellt, dass der Einsatz einer Spritze um ein Vakuum anzulegen durchaus vorteilhaft ist, da oftmals nicht schnell genug irgendwelche Pumpen vorhanden sind, mit denen gegebenenfalls ein Vakuum maschinell hergestellt werden kann.

Allerdings kann in diesem Zusammenhang vorgesehen sein, das der Anschlusskörper einen hülsenförmigen Ansatz, also einen weiteren Anschluss für z. B. den Schlauch einer Pumpe aufweist. Dieser hülsenförmige Ansatz ist üblicherweise verschlossen, da ansonsten mit Hilfe der Spritze ein Vakuum nicht gezogen werden könnte, oder aber es ist vorgesehen, dass der Schlauch zwischen Ansatz und Pumpe eine Klemme zum Verschließen besitzt.

Anhand der Zeichnungen wird die Erfindung beispielhaft näher erläutert.
- Figur 1: zeigt den Trokar mit über die Hülse überstehender Dornspitze;
- Figur 2: zeigt die Gehäuseeinrichtung im Schnitt in der Stellung des Dorns gemäß Figur 1;
- Figur 3: zeigt den Arretierstift mit Feder in der Stellung gemäß Figur 2;
- Figur 4: zeigt den Trokar in einer Stellung, in der der Dorn durch die Hülse am stirnseitigen Ende abgedeckt ist;
- Figur 5: zeigt die Gehäuseeinrichtung in einer Stellung des Dorns gemäß Figur 4;
- Figur 6: zeigt den Anschlusskörper einschließlich der Hülse mit einsitzendem Dom;
- Figur 7: zeigt den Anschlusskörper gemäß Figur 6 ohne Dorn;
- Figur 8: zeigt den Anschlusskörper einschließlich der Hülse mit angesetzter Spritze;
- Figur 9: zeigt eine Schnittdarstellung gemäß Figur 8 in vergrößerter Darstellung.

Der insgesamt mit 1 bezeichnete Trokar umfasst die mit 10 bezeichnete Gehäuseeinrichtung sowie den mit 30 bezeichneten Anschlusskörper, der die Hülse 31 aufnimmt. In dem Gehäuse 11 der Gehäuseeinrichtung 10 befindet sich der mit 20 bezeichnete Dorn. Der Dorn 20 besitzt an seinem vorderen distalen Ende eine Spitze 21, wie dies üblicherweise bekannt ist. Am hinteren Ende zeigt der Dorn einen Teller 22, wobei die Gehäuseeinrichtung 10 die insgesamt mit 15 bezeichnete Arretiereinrichtung aufweist.

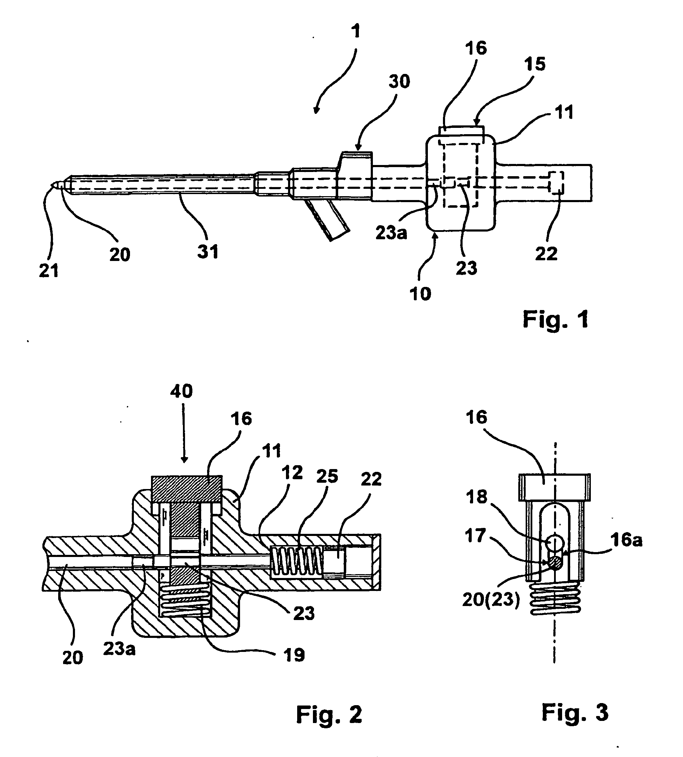

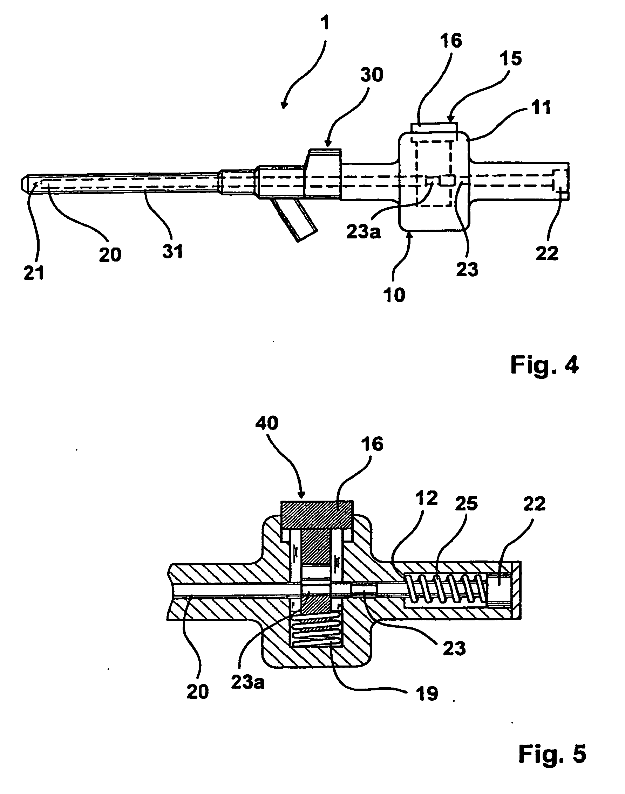

Erkennbar ist insbesondere aus Figur 2, dass der Dorn 20 in der Stellung, in der er über die Hülse 31 übersteht (Figur 1), von dem Arretierstift 16 der Arretiereinrichtung in der Nut 23 erfasst wird und hierdurch arretiert ist, wie sich dies unmittelbar in Anschauung auch von Figur 3 ergibt. Dies insofern, als dort erkennbar ist, dass die Nut 23 in dem unteren Durchmesser 17 des Schlitzes 16a des Arretierstiftes einliegt, wobei der Durchmesser 17 vom Querschnitt geringer ist als der obere Durchmesser 18 in dem Schlitz 16a, der in etwa dem Querschnitt des Dorns außerhalb der Nuten entspricht. Wird nun der Arretierstift 16 in Richtung des Pfeils 40 gedrückt, dann gelangt der Dorn 20 mit seiner Nut 23 in den Bereich des Durchmessers 18 des Arretierstiftes 16, so dass dann, wenn sich der Dorn 20 in diesem Bereich des Durchmessers 18 befindet, der Dorn 20 axial beweglich ist. Erkennbar steht der Dorn 20 unter der Last einer Dornfeder 25, die sich einerseits am Teller 22 abstützt und andererseits am Bund 12 des Gehäuses 11. Der Dorn stehet in der Stellung gemäß Figur 1 unter der Last der Feder 25, so dass dann, wenn der Arretierstift 16 in Richtung des Pfeils 40 entgegen der Kraft der Feder 19 verschoben wird, der Dorn aufgrund der Federkraft der Feder 25 in die Endstellung gemäß Figur 4 bzw. Figur 5 gelangt, und hierbei der Arretierstift in Eingriff mit der Nut 23a des Dorns 20 gelangt, wie dies in Figur 5 dargestellt ist. In dieser Stellung des Dorns steht die Hülse 31 über den Dorn über (Figur 4).

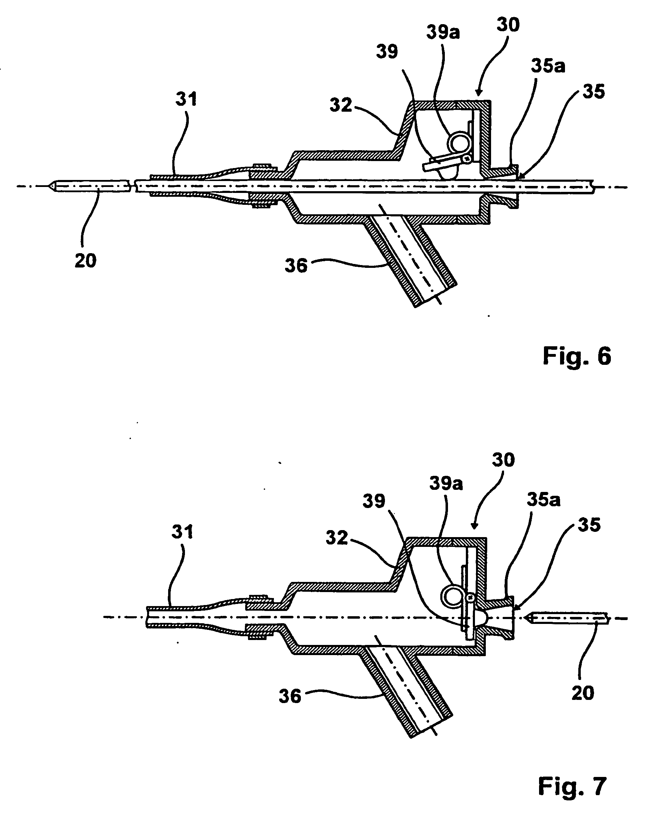

Gegenstand der Erfindung ist ebenfalls die Ausbildung des Anschlusskörpers 30. In diesem Zusammenhang wird im Folgenden auf die Figuren 6 bis 9 Bezug genommen.

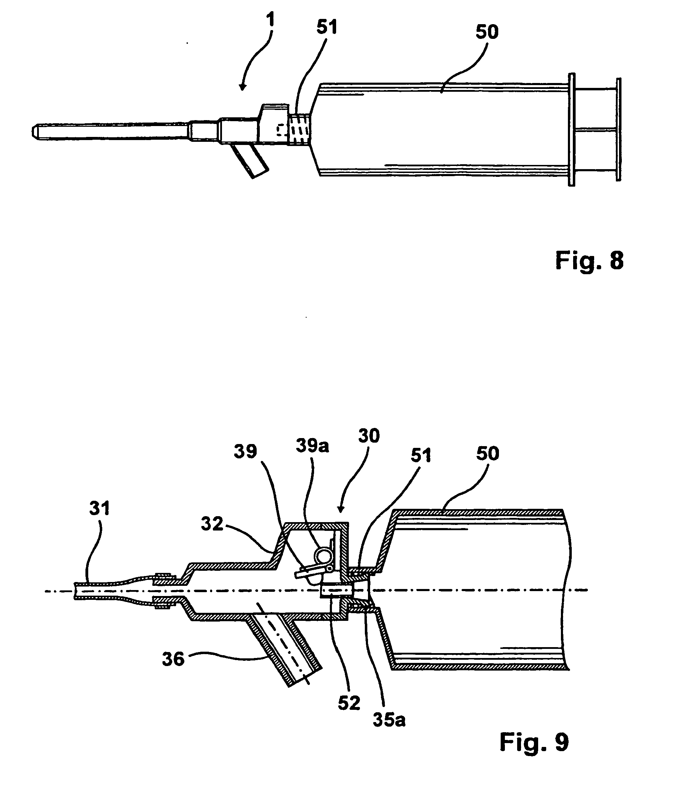

Der Anschlusskörper 30 weist endseitig die Hülse 31 auf, innerhalb derer der Dorn relativ beweglich ist. Der Anschlusskörper 30 besitzt das Gehäuse 32, wobei das Gehäuse 32 zwei Anschlüsse 35 und 36 mit entsprechenden Durchlässen aufweist. Der Durchlass 35 weist einen Stutzen 35a auf, wohingegen der Durchlass 36 beispielsweise dem Anschluss eines Schlauches dient und ansonsten durch einen Deckel (nicht dargestellt) verschließbar ist. Der Stutzen 35a dient der Aufnahme einer Spritze, wie sich dies in Anschauung der Figuren 8 und 9 ergibt. Die Spritze 50 besitzt einen Ansatz 51, der auf den Anschlussstutzen 35a aufgeschraubt wird. Im Inneren des Ansatzes 51 weist die Spritze einen hohlen Anschlussdorn 52 auf, der in das Innere des Anschlusskörpers 30 ragt, und hierbei die federbelastete Klappe 39 in die Stellung gemäß Figur 9 stößt. In der Stellung gemäß Figur 9 besteht nunmehr die Möglichkeit, mit Hilfe der Spritze 50 ein Vakuum im Brustraum zu ziehen. Wird die Spritze entfernt, schließt die Klappe 39 aufgrund der Feder 39a, wobei dann beispielsweise durch Anschluss eines Schlauches mit einer Vakuumpumpe an den Anschluss 36 über diesen Durchlass im Anschluss das Vakuum gezogen werden kann.

## Patentansprüche

1. Torax-Trokar (1), umfassend einen in einer Gehäuseeinrichtung (10) axial beweglichen Dorn (20), wobei der Dorn (20) eine abnehmbare Hülse (31) aufweist, wobei der Dorn (20) relativ zu der Hülse (31) in mindestens einer Stellung in der Gehäuseeinrichtung (10) durch eine Arretiereinrichtung (15) arretierbar ist, wobei in einer ersten arretierten Stellung der Dorn (20) mit seiner Spitze (21) über die Hülse (31) übersteht, und wobei in einer zweiten Stellung der Dorn (20) durch die Hülse (31) überstehend abgedeckt ist,
**dadurch gekennzeichnet,**
**dass** der Dorn (20) in der zweiten zurückgezogenen Stellung in der Gehäuseeinrichtung (10) durch die Arretiereinrichtung (15) arretierbar ist.

2. Torax-Trokar nach Anspruch 1,
**dadurch gekennzeichnet,**
**dass** die Hülse (31) aus elastisch nachgiebigem Material ausgebildet ist.

3. Torax-Trokar nach einem der voranstehenden Ansprüche,
**dadurch gekennzeichnet,**
**dass** die Gehäuseeinrichtung (10) einen Arretierstift (16) umfasst, der einen in Längsrichtung verlaufenden Schlitz (16a) aufweist, wobei der Schlitz (16a) zwei übereinander angeordnete unterschiedliche Durchmesser (17, 18) besitzt, wobei der Dorn (20) zwei beabstandet zueinander angeordnete Nuten (23, 23a) zur Erzeugung eines zum Ausgangsdurchmesser des Dorns (20) unterschiedlich reduzierten Durchmessers aufweisen, wobei der im Schlitz (16a) angeordnete untere Durchmesser (17) mit dem Durchmesser oder dem Querschnitt der Nuten (23, 23a) korreliert und der Ausgangsdurchmesser des Dorns (20) mit dem oberen Durchmesser oder Querschnitt im Schlitz (16a) des Arretierstiftes (16).

4. Torax-Trokar nach einem der voranstehenden Ansprüche,
**dadurch gekennzeichnet,**
**dass** der Arretierstift (16) in dem Gehäuse (11) der Gehäuseeinrichtung (10) unter der Last einer Feder (19) steht.

5. Torax-Trokar nach einem der voranstehenden Ansprüche,
**dadurch gekennzeichnet,**
**dass** der Dorn (20) in dem Gehäuse (11) der Gehäuseeinrichtung (10) entgegen der Kraft einer Dornfeder (25) aus dem Gehäuse (11) der Gehäuseeinrichtung (10) herausziehbar ist.

6. Torax-Trokar nach einem der voranstehenden Ansprüche,
**dadurch gekennzeichnet,**
**dass** die Dornfeder (25) sich einerseits axial an einem Bund (12) in dem Gehäuse (11) der Gehäuseeinrichtung (10) und anderseits an einem endseitig am Dorn (20) angeordneten Teller (22) abstützt.

7. Torax-Trokar nach einem der voranstehenden Ansprüche,
**dadurch gekennzeichnet,**
**dass** die Hülse (31) an dem der Gehäuseeinrichtung (10) zugewandten Ende einen Anschlusskörper (30) mit mindestens einem Anschluss (35, 36) mit entsprechendem Durchlass aufweist, wobei der Anschluss (35) durch eine schwenkbare Klappe (39) verschließbar ist.

8. Torax-Trokar nach Anspruch 7,
**dadurch gekennzeichnet,**
**dass** die Klappe (39) federbelastet (39a) ist.

9. Torax-Trokar nach Anspruch 7 oder 8,
**dadurch gekennzeichnet,**
**dass** der Anschlusskörper (30) im Bereich des Durchlasses (35) einen Stutzen (35a) zur Aufnahme einer Spritze (50) aufweist.

10. System bestehend aus einer Spritze (50) und dem Torax-Trokar nach Anspruch 9,
**dadurch gekennzeichnet,**
**dass** die Spritze (50) ein Mundstück (51) zur Aufnahme durch den Stutzen (35a) besitzt.

11. System nach Anspruch 10,
**dadurch gekennzeichnet,**
**dass** das Mundstück (51) einen hohlen Anschlussdorn (52) aufweist, der bis in den Anschlusskörper (30) ragt und die Klappe (39) aufstößt.

12. Torax-Trokar nach einem der Ansprüche 7 bis 10,
**dadurch gekennzeichnet,**
**dass** der Anschlusskörper (30) einen hülsenförmigen Anschluss (36) für einen Schlauch aufweist

## Claims

1. Thoracic trocar (1) comprising a spike (20) axially movable in a housing apparatus (10), wherein the spike (20) comprises a detachable sleeve (31), wherein the spike (20) is lockable with respect to the sleeve (31) in at least one position in the housing apparatus (10) by a locking device (15), wherein, in a first locked position, the spike (20) with its tip (21) protrudes beyond the sleeve (31) and wherein, in a second position, the spike (20) is covered by the sleeve (31) in a protruding manner beyond the sleeve,
**characterized in that**
the spike (20) is lockable by the locking device (15) in the second retracted position in the housing apparatus (10).

2. Thoracic trocar according to claim 1,
**characterized in that**
the sleeve (31) is made from elastic resilient material.

3. Thoracic trocar according to any of the preceding claims,
chararcterized in that
the housing apparatus (10) comprises a locking pin (16) comprising a slit (16a) running along a longitudinal direction, wherein the slit (16a) has two different diameters (17, 18) arranged one above the other, wherein the spike (20) comprises two grooves (23, 23a) arranged in a spaced manner to each other for creating a diameter differently reduced with respect to the initial diameter of the spike (20), wherein the lower diameter (17) arranged in the slit (16a) correlates with the diameter or the cross-section of the grooves (23, 23a) and the initial diameter of the spike (20) correlates with the upper diameter or cross-section in the slit (16a) of the locking pin (16).

4. Thoracic trocar according to any of the preceding claims,
**characterized in that**
the locking pin (16) in the housing (11) of the housing apparatus (10) is biased by a spring (19).

5. Thoracic trocar according to any of the preceding claims,
**characterized in that**
the spike (20) in the housing (11) of the housing apparatus (10) is extensible out of the housing (11) of the housing apparatus (10) against the force of a spike spring (25).

6. Thoracic trocar according to any of the preceding claims,
**characterized in that**
the spike spring (25) axially supports oneself at a shoulder (12) in the housing (11) of the housing apparatus (10) on the one side and at a disk (22) arranged at an end side of the spike (20) on the other side.

7. Thoracic trocar according to any of the preceding claims,
chararcterized in that
the sleeve (31) comprises, at the end side facing the housing apparatus (10), a connection body (30) having at least one connection point (35, 36) comprising an appropriate passage, wherein the connection point (35) is closable by a pivotable flap (39).

8. Thoracic trocar according to claim 7,
**characterized in that**
the flap (39) is spring-loaded (39a).

9. Thoracic trocar according to claim 7 or 8,
**characterized in that**
the connection body (30) comprises a connecting piece (35a) for accommodating a syringe (50) in the region of the passage (35).

10. System consisting of a syringe (50) and the thoracic trocar according to claim 9,
**characterized in that**
the syringe (50) has a mouthpiece (51) for being accommodated by the connecting piece (35a).

11. System according to claim 10,
**characterized in that**
the mouthpiece (51) comprises a hollow connection spike (52) protruding into the connection body (30) and pushing open the flap (39).

12. Thoracic trocar according to any of claims 7 to 10,
chararcterized in that
the connection body (30) comprises a sleeve-shaped connection point (36) for a hose.

## Revendications

1. Trocart thoracique (1), comprenant un poinçon (20) mobile en direction axiale dans un dispositif à boîtier (10), ledit poinçon (20) comportant un manchon (31) amovible, ledit poinçon (20) pouvant être bloqué par un système d'arrêt (15) dans le dispositif à boîtier (10), dans au moins une position par rapport au manchon (31), ledit poinçon (20) dépassant du manchon (31) par sa pointe (21) dans une première position de blocage, et ledit poinçon (20) étant recouvert par le manchon (31) en saillie dans une deuxième position,
**caractérisé**
**en ce que** ledit poinçon (20) peut être bloqué par le système d'arrêt (15) en deuxième position de retrait dans le dispositif à boîtier (10).

2. Trocart thoracique selon la revendication 1,
**caractérisé**
**en ce que** le manchon (31) est constitué d'un matériau élastiquement flexible.

3. Trocart thoracique selon l'une des revendications précédentes,
**caractérisé**
**en ce que** le dispositif à boîtier (10) comprend une goupille d'arrêt (16) qui comporte une fente (16a) s'étendant en direction longitudinale, ladite fente (16a) présentant deux diamètres (17, 18) différents superposés, le poinçon (20) comportant deux rainures (23, 23a) espacées l'une de l'autre pour obtenir une réduction différenciée de diamètre par rapport au diamètre initial du poinçon (20), le diamètre (17) inférieur présent dans la fente (16a) correspondant au diamètre ou à la section transversale des rainures (23, 23a) et le diamètre initial du poinçon (20) correspondant au diamètre supérieur ou à la section transversale de la fente (16a) de la goupille d'arrêt (16).

4. Trocart thoracique selon l'une des revendications précédentes,
**caractérisé**
**en ce que** la goupille d'arrêt (16) est placée sous la contrainte d'un ressort (19) dans le boîtier (11) du dispositif à boîtier (10).

5. Trocart thoracique selon l'une des revendications précédentes,
**caractérisé**
**en ce que** le poinçon (20) disposé dans le boîtier (11) du dispositif à boîtier (10) peut être retiré du boîtier (11) du dispositif à boîtier (10), contre la force d'un ressort de poinçon (25).

6. Trocart thoracique selon l'une des revendications précédentes,
**caractérisé**
**en ce que** le ressort de poinçon (25) prend appui axialement contre un épaulement (12) dans le boîtier (11) du dispositif à boîtier (10), d'une part, et prend appui d'autre part contre une plaque (22) disposée à l'extrémité du poinçon (20).

7. Trocart thoracique selon l'une des revendications précédentes,
**caractérisé**
**en ce qu'**à son extrémité adjacente au dispositif à boîtier (10), le manchon (31) comporte un corps de connexion (30) ayant au moins un raccord (35, 36) avec un passage correspondant, ledit raccord (35) étant obturable par un volet (39) pivotant.

8. Trocart thoracique selon la revendication 7,
**caractérisé**
**en ce que** le volet (39) est contraint par un ressort (39a).

9. Trocart thoracique selon la revendication 7 ou la revendication 8,
**caractérisé**
**en ce que** le corps de connexion (30) comporte une tubulure (35a) pour la réception d'une seringue (50) au niveau du passage (35).

10. Système composé de la seringue (50) et du trocart thoracique selon la revendication 9,
**caractérisé**
**en ce que** la seringue (50) comporte un embout (51) pour le logement dans la tubulure (35a).

11. Système selon la revendication 10, **caractérisé en ce que** l'embout (51) comprend un dôme de connexion (52) creux qui pénètre dans le corps de connexion (30) et ouvre le volet (39).

12. Trocart thoracique selon l'une des revendications 7 à 10, **caractérisé en ce que** le corps de connexion (30) comporte un raccord (36) en forme de manchon pour un tuyau.
